# EUROPÄISCHE PATENTSCHRIFT

(11) **EP 0 378 082 B1**
(45) Veröffentlichungstag und Bekanntmachung des Hinweises auf die Patenterteilung: **08.06.1994**
(21) Anmeldenummer: 90100058.8
(22) Anmeldetag: 03.01.1990
(51) Int. Cl.: C07D 249/12

(54) **1-Hydroxy-1,2,4-triazole**
1-Hydroxy-1,2,4-triazoles
Hydroxy-1 triazoles-1,2,4

(30) Priorität: 07.01.1989 DE 3900347
(43) Veröffentlichungstag der Anmeldung: 18.07.1990
(73) Patentinhaber: BASF Aktiengesellschaft, 67063 Ludwigshafen (DE)
(72) Erfinder: Baus, Ulf, Dr., D-6915 Dossenheim (DE); Reuther, Wolfgang, Dr., D-6900 Heidelberg (DE)

(56) Entgegenhaltungen:
- DD-A- 59 288
- DE-A- 3 820 738
- DE-A- 3 820 739
- CHEMICAL ABSTRACTS, Band 105, Nr. 17, 27. Oktober 1986, Columbus, Ohio, USA, Seite 638, Spalte 1, Zusammenfassung-Nr. 152441u; JOVANOVIC M.V.: "Synthesis of the first monosubstituted 1,2,4-triazine di-N-oxide. Carbon-13 NMR of 1,2,4-triazine N-oxides and use of hydrogen/deuterium isotope shifts for the assignments of some dihydro-1,2,4-triazine tautomers"
- CHEMICAL ABSTRACTS, Band 107, Nr. 21, 23. November 1987, Columbus, Ohio, USA, Seite 88, Spalte 2, Zusammenfassung-Nr. 191136j; WICKINGS E.J. et al.: "Non-steroidal inhibition of granulosa cell aromatase activity in vitro"

## Beschreibung

Die vorliegende Erfindung betrifft neue 1-Hydroxy-1,2,4-triazole und Verfahren zu deren Herstellung.

Aus der DE-A-22 01 063 und der DE-A-23 24 010 sind wertvolle in 1-Position substituierte 1,2,4-Triazole mit biologischer Aktivität bekannt.

Aus der DE-A-38 20 738 und der DE-A-38 20 739 sind Verfahren zur Herstellung von 1-Hydroxypyrazolen mit Peroxogruppen enthaltenden Verbindungen bekannt.

Der vorliegenden Erfindung lag daher die Aufgabe zugrunde, neue Zwischenprodukte zu finden, die einen einfachen Zugang zu neuen in 1-Position substituierten 1,2,4-Triazolen ermöglichen (mittlerweile beschrieben z.B. in EP-A-383 197, EP-A-385 371 und EP-A-396 078).

Demgemäß wurden neue 1-Hydroxy-1,2,4-triazole der allgemeinen Formel I 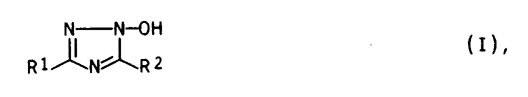
in der
- R¹ und R²: unabhängig voneinander Wasserstoff, Alkyl, Halogen, gegebenenfalls substituiertes Aryl oder gemeinsam eine Alkylenkette bedeuten,
gefunden sowie Verfahren zu deren Herstellung.

Die Substituenten R¹ und R² der 1-Hydroxy-1,2,4-triazole I, gegebenenfalls Salze als Zwischenprodukte III sowie deren Vorprodukte, die 1-H-1,2,4-Triazole II, haben unabhängig voneinander folgende Bedeutungen:
- Wasserstoff,
- Alkyl wie C₁-C₂₀-Alkyl, bevorzugt C₁-C₈-Alkyl, besonders bevorzugt C₁-C₄-Alkyl wie Methyl, Ethyl, n-Propyl, iso-Propyl, n-Butyl, iso-Butyl, sec.-Butyl und tert.-Butyl,
- Halogen, wie Fluor, Chlor, Brom und Iod, bevorzugt Chlor, Brom und Iod, besonders bevorzugt Chlor,
- Aryl wie Phenyl, 1-Naphthyl, 2-Naphthyl, 1-Anthranyl, 2-Anthranyl und 9-Anthranyl, bevorzugt Phenyl, 1-Naphthyl und 2-Naphthyl, besonders bevorzugt Phenyl,
- durch Alkyl und/oder Halogen ein- bis dreifach substituiertes Aryl, bevorzugt durch C₁-C₈-Alkyl und/oder Fluor, Chlor, Brom oder Iod ein- bis dreifach substituiertes Phenyl, besonders bevorzugt durch C₁-C₄-Alkyl und/oder Fluor oder Chlor ein- bis dreifach substituiertes Phenyl wie 2-Fluorphenyl, 4-Fluorphenyl, 2-Chlorphenyl, 4-Chlorphenyl, 2,4-Difluorphenyl, 2,4-Dichlorphenyl, 2-Chlor-4-fluorphenyl, 2,4,6-Trifluorphenyl und 2,4,6-Trichlorphenyl.

Die Substituenten R¹ und R² in den Verbindungen I, II und III können auch gemeinsam für eine Alkylenkette stehen, die gegebenenfalls durch Alkyl, Halogen und/oder gegebenenfalls substituiertes Aryl substituiert sein kann. Die bevorzugten Substituenten der Alkylenkette sind analog denjenigen, die zuvor genannt wurden. Die Alkylenkette besteht aus 4- oder 5-Kettengliedern, also -(CH₂)₄- oder -(CH₂)₅-, bevorzugt ist -(CH₂)₅-.

In den Verbindungen III bedeutet Me^{⊕} ein Alkali- oder Erdalkalikation, bevorzugt Lithium, Natrium, Kalium, Magnesium und Calcium, besonders bevorzugt Lithium, Natrium und Kalium.

Bevorzugte 1-Hydroxy-1,2,4-triazole I und bevorzugte 1-H-1,2,4-Triazole II sind:

| 1-Hydroxy-1,2,4-triazole I | 1-H-1,2,4-Triazole II |
|---|---|
| 1-Hydroxy-1,2,4-triazol | 1-H-1,2,4-Triazol |
| 1-Hydroxy-3-methyl-1,2,4-triazol | 1-H-3-methyl-1,2,4-triazol |
| 1-Hydroxy-5-methyl-1,2,4-triazol | 1-H-5-methyl-1,2,4-triazol |
| 1-Hydroxy-3,5-dimethyl-1,2,4-triazol | 1-H-3,5-dimethyl-1,2,4-triazol |
| 1-Hydroxy-3-ethyl-1,2,4-triazol | 1-H-3-ethyl-1,2,4-triazol |
| 1-Hydroxy-5-ethyl-1,2,4-triazol | 1-H-5-ethyl-1,2,4-triazol |
| 1-Hydroxy-3,5-diethyl-1,2,4-triazol | 1-H-3,5-diethyl-1,2,4-triazol |
| 1-Hydroxy-3-ethyl-5-methyl-1,2,4-triazol | 1-H-3-ethyl-5-methyl-1,2,4-triazol |
| 1-Hydroxy-5-ethyl-3-methyl-1,2,4-triazol | 1-H-5-ethyl-3-methyl-1,2,4-triazol |
| 1-Hydroxy-3-chlor-1,2,4-triazol | 1-H-3-chlor-1,2,4-triazol |
| 1-Hydroxy-5-chlor-1,2,4-triazol | 1-H-5-chlor-1,2,4-triazol |
| 1-Hydroxy-3,5-dichlor-1,2,4-triazol | 1-H-3,5-dichlor-1,2,4-triazol |
| 1-Hydroxy-3,5-diphenyl-1,2,4-triazol | 1-H-3,5-diphenyl-1,2,4-triazol |
| 1-Hydroxy-3-(4-fluorphenyl)-1,2-triazol | 1-H-3-(4-fluorphenyl)-1,2,4-triazol |

Besonders bevorzugt wird 1-Hydroxy-1,2,4-triazol als Verbindung I und 1-H-1,2,4-Triazol als Verbindung II.

Die 1-Hydroxy-1,2,4-triazole I sind nach folgenden Methoden erhältlich:
Die Umsetzung erfolgt zwischen einem 1-H-1,2,4-Triazol II und einer Verbindung mit Peroxogruppen bei Temperaturen von -20°C bis +150°C, gegebenenfalls in Gegenwart eines Agenzes, das unter Salzbildung über die Verbindungen III zu den 1-Hydroxy-1,2,4-triazolen I nach folgender Reaktionsgleichung führt:

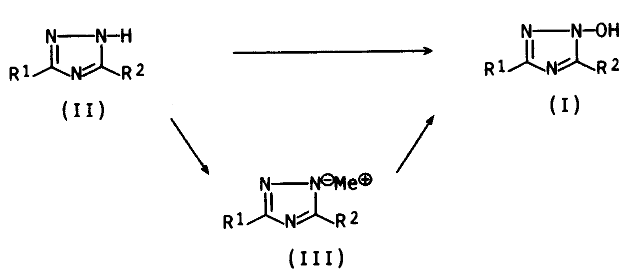

Für den Fall, daß man die 1-H-1,2,4-Triazole II mit Peroxogruppen tragenden Verbindungen ohne Zusatz eines salzbildenden Agenzes umsetzt, verfährt man wie folgt:
1 bis 3 eq (Mol-Äquivalent) 1-H-1,2,4-Triazol II werden in einem Lösungsmittel wie Wasser, Wasser/Aceton-Mischung, Tetrahydrofuran, Diglyme, Methylchlorid oder Chloroform mit 1 eq einer Peroxocarbonsäure, vorzugsweise m-Chlorperbenzoesäure versetzt. Die Reaktionstemperatur liegt zwischen 0 und 50°C, vorzugsweise bei Raumtemperatur.

Für den Fall, daß man die 1-H-1,2,4-Triazole mit Peroxogruppen tragenden Verbindungen unter Zusatz eines salzbildenden Agenzes umsetzt, verfährt man wie folgt:
a) 1 bis 10 eq 1-H-1,2,4-Triazol II in einem inerten Lösungsmittel wie Diglyme, Tetrahydrofuran oder Diethylether mit einer metallorganischen Verbindung, einer Alkalimetallsuspension oder einem Hydrid metalliert, anschließend mit 1 eq Dibenzoylperoxid versetzt. Man läßt mehrere Tage bei Raumtemperatur rühren.
b) 1 bis 3 eq 1-H-1,2,4-Triazol II werden in Wasser mit einem Hydroxid, einem Carbonat oder einem Hydrocarbonat metalliert, anschließend mit 1 eq Peroxocarbonsäure versetzt und über Nacht gerührt. Statt der Peroxocarbonsäure kann man auch das Alkali- oder Erdalkalisalz der Peroxocarbonsäure verwenden und z.B. in fester Form hinzudosieren.

Als salzbildende Agentien eignen sich metallorganische Verbindungen z.B. Metallalkyle wie n-Butyllithium, tert.-Butyllithium und Methyllithium, Metallaryle wie Phenyllithium, Alkalimetallsuspensionen, wie Natrium in Toluol oder Kalium in Toluol, Hydride, z.B. Alkalihydride wie Lithiumhydrid, Natriumhydrid und Kaliumhydrid, Erdalkalihydride wie Calciumhydrid, bevorzugt Natriumhydrid, Hydroxide, z.B. Alkalihydroxide wie Lithiumhydroxid, Natriumhydroxid und Kaliumhydroxid, Erdalkalihydroxide wie Calciumhydroxid und Magnesiumhydroxid, Carbonate, z.B. Alkalicarbonate wie Lithiumcarbonat, Natriumcarbonat und Kaliumcarbonat, Erdalkalicarbonate wie Calciumcarbonat und Magnesiumcarbonat, Hydrogencarbonate, z.B. Natriumhydrogencarbonat.

Diese Reaktionen können bevorzugt auch in Gegenwart eines Lösungsmittels durchgeführt werden. Bei der Verwendung von metallorganischen Verbindungen oder Hydriden eignen sich Ether wie Diethylether, Methyl-butylether, Tetrahydrofuran und Dioxan, Glykolether wie Diglyme, Triglyme, aliphatische Kohlenwasserstoffe wie Pentan, Hexan, Petrolether, Cyclohexan, aromatische Kohlenwasserstoffe wie Benzol, Toluol und die Xylole oder deren Gemische.

Bei der Verwendung von Hydroxiden, Carbonaten oder Hydrogencarbonaten eignen sich Wasser, Alkohole, wie Methanol, Ethanol, n-Propanol, iso-Propanol und die Butanole, Ketone, wie Aceton und Diethylketon oder deren Gemische, bevorzugt Wasser.

Als Peroxogruppen tragende Verbindungen eignen sich organische Peroxide, z.B. Dialkylperoxide, Alkylarylperoxide, Diarylperoxide, Diacylperoxide wie Diacetylperoxid, Dipropionylperoxid und Dibenzoylperoxid, bevorzugt Dibenzoylperoxid; Peroxosäuren, z.B. Peroxosulfonsäuren wie p-Toluolperoxosulfonsäure, Toluolperoxosulfonsäure, p-Bromtoluolperoxosulfonsäure und Methylperoxosulfonsäure, bevorzugt p-Toluolsulfonsäure, Peroxocarbonsäuren wie Peroxoessigsäure, Peroxobenzoesäure, m-Chlorperbenzoesäure, Peroxopropionsäure, Peroxobuttersäure, Peroxomaleinsäure, Monoperoxobernsteinsäure und Monoperoxophthalsäure, bevorzugt Monoperoxophthalsäure.

Die 1-Hydroxy-1,2,4-triazole I eignen sich als Zwischenprodukte für biologisch aktive Substanzen wie Fungizide, Wachstumsregulatoren und Biozide.

### Beispiel

103,5 g (1,5 mol) 1-H-1,2,4-Triazol wurden in 1344 g (12 mol) 50 %igem wäßrigem Kaliumhydroxid gelöst. Unter Eiskühlung wurden 340 g (3 mol) 30 %iges H₂O₂, portionsweise 555 g (3,75 mol) Phthalsäureanhydrid zugegeben und 2 Stunden bei Raumtemperatur (20 bis 30°C) gerührt. Anschließend wurde mit ca. 35 %iger Schwefelsäure auf einen pH-Wert <1,5 angesäuert, der entstandene Niederschlag abgesaugt und das Filtrat durch quantitative HPLC-Messung untersucht. Man erhielt 19 g (15 %) das wie üblich aufgearbeitet wurde; Fp.: 132°C.

## Patentansprüche

1. 1-Hydroxy-1,2,4-triazole der allgemeinen Formel I 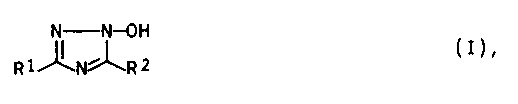 in der
R¹ und R² unabhängig voneinander Wasserstoff, Alkyl, Halogen, gegebenenfalls substituiertes Aryl oder gemeinsam eine Alkylenkette bedeuten.

2. 1-Hydroxy-1,2,4-triazole der allgemeinen Formel I nach Anspruch 1, in der R¹ und R² unabhängig voneinander Wasserstoff, C₁- bis C₂₀-Alkyl, Fluor, Chlor, Brom, Phenyl, 1-Naphthyl, 2-Naphthyl, durch C₁- bis C₈-Alkyl und/oder Fluor, Chlor, Brom oder Iod ein- bis dreifach substituiertes Phenyl oder gemeinsam -(CH₂)₄- oder -(CH₂)₅.

3. 1-Hydroxy-1,2,4-triazole der allgemeinen Formel I nach Anspruch 1, in der R¹ und R² unabhängig voneinander Wasserstoff, C₁- bis C₈-Alkyl, Chlor, Phenyl, durch C₁- bis C₄-Alkyl und/oder Fluor oder Chlor, ein- bis dreifach substituiertes Phenyl oder gemeinsam -(CH₂)₄-.

4. Verfahren zur Herstellung von 1-Hydroxy-1,2,4-triazolen der allgemeinen Formel I nach Anspruch 1, dadurch gekennzeichnet, daß man 1-H-1,2,4-Triazole der allgemeinen Formel II 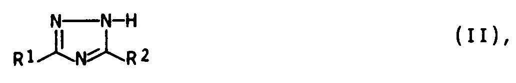 in der R¹ und R² die oben genannten Bedeutungen haben, mit einer Verbindung mit Peroxogruppen, gegebenenfalls in Gegenwart eines Agenzes, das unter Salzbildung über die Verbindungen der allgemeinen Formel III 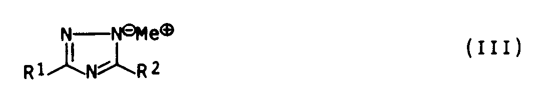 in der R¹ und R² die oben genannten Bedeutungen haben und Me^{⊕} für ein Alkali- oder Erdalkalimetallkation steht, führt, umsetzt.

## Claims

1. A 1-hydroxy-1,2,4-triazole of the general formula I 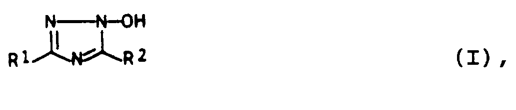 where
R¹ and R² are singly independently of one another hydrogen, alkyl, halogen or substituted or unsubstituted aryl or together an alkylene chain.

2. A 1-hydroxy-1,2,4-triazole of the general formula I as claimed in claim 1, wherein R¹ and R² are each independently of the other hydrogen, C₁-C₂₀-alkyl, fluorine, chlorine, bromine, phenyl, 1-naphthyl, 2-naphthyl, or C₁-C₈-alkyl and/or fluorine-, chlorine-, bromine-, or iodine-monosubstituted, -disubstituted or - trisubstituted phenyl, or together -(CH₂)₄- or -(CH₂)₅-.

3. A 1-hydroxy-1,2,4-triazole of the general formula I as claimed in claim 1, wherein R¹ and R² are each independently of the other hydrogen, C₁-C₈-alkyl, chlorine, phenyl or C₁-C₄-alkyl and/or fluorine- or chlorine- monosubstituted, -disubstituted or -trisubstituted phenyl or together -(CH₂)₄-.

4. A process for preparing a 1-hydroxy-1,2,4-triazole of the general formula I as claimed in claim 1, which comprises reacting a 1-H-1,2,4-triazole of the general formula II 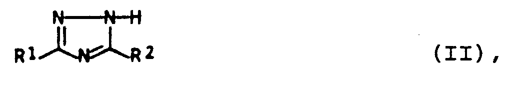 where R¹ and R² are each as defined above, with a peroxy compound in the presence or absence of an agent which by salt formation leads via a compound of the general formula III 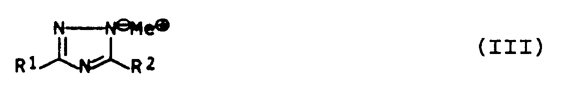 where R¹ and R² are as defined above and Me^{⊕} is an alkali metal or alkaline earth metal cation.

## Revendications

1. 1-Hydroxy-1,2,4-triazoles de la formule générale I 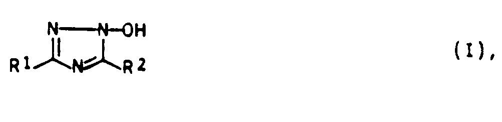 dans laquelle
R¹ et R² représentent, chacun indépendamment l'un de l'autre, un atome d'hydrogène, un atome d'halogène, un radical alkyle, un radical aryle éventuellement substitué, ou forment ensemble une chaîne alkylénique.

2. 1-Hydroxy-1,2,4-triazoles de la formule générale I suivant la revendication 1, caractérisés en ce que R¹ et R² représentent, chacun indépendamment l'un de l'autre, un atome d'hydrogène, un radical alkyle en C₁ à C₂₀, un atome de fluor, de chlore, de brome, un radical phényle, 1-naphtyle, 2-naphtyle, un radical phényle d' une à trois fois substitué par des radicaux alkyle en C₁ à C₈ et/ou des atomes de fluor, de chlore, de brome ou d'iode, ou forment ensemble le radical -(CH₂)₄- ou -(CH₂)₅.

3. 1-Hydroxy-1,2,4-triazoles de la formule générale I suivant la revendication 1, caractérisés en ce que R¹ et R² représentent, chacun indépendamment l'un de l'autre, un atome d'hydrogène, un radical alkyle en C₁ à C₈, un atome de chlore, un radical phényle, un radical phényle substitué de 1 à 3 fois par des radicaux alkyle en C₁ à C₄ et/ou des atomes de fluor ou de chlore, ou forment ensemble le radical -(CH₂)₄-.

4. Procédé de préparation de 1-hydroxy-1,2,4-triazoles de la formule générale I suivant la revendication 1, caractérisé en ce que l'on fait réagir des 1-H-1,2,4-triazoles de la formule générale II 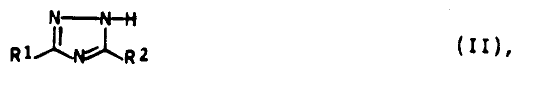 dans laquelle R¹ et R² possèdent les significations qui leur ont été attribuées ci-dessus, avec un composé comportant des radicaux peroxo, éventuellement en présence d'un agent qui conduit, sous formation de sel et en passant par les composés de la formule générale III 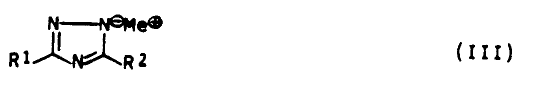 dans laquelle R¹ et R² possèdent les significations qui leur ont été attribuées ci-dessus et Me^{⊕} représente un cation de métal alcalin ou de métal alcalino-terreux.
